# EUROPEAN PATENT SPECIFICATION

(11) **EP 1 779 802 B1**
(45) Date of publication and mention of the grant of the patent: **06.04.2016**
(21) Application number: 06255592.5
(22) Date of filing: 31.10.2006
(51) Int. Cl.: A61B 8/12

(54) **Controlling direction of ultrasound imaging catheter**
Steuerung der Richtung eines bildgebenden Katheter
Contrôle de la direction d'un cathéter d'imagerie ultrasonique

(30) Priority: 01.11.2005 US 264221
(43) Date of publication of application: 02.05.2007
(73) Proprietor: Biosense Webster, Inc., Diamond Bar, CA 91765 (US)
(72) Inventor: Altmann, Andres Claudio, Haifa 34614 (IL); Ephrath, Yaron, Karkur 37501 (IL); Govari, Assaf, 34400 Haifa (IL)
(74) Representative: Tunstall, Christopher Stephen

(56) References cited:
- WO-A-01/89405
- WO-A-97/29682
- WO-A-99/27861
- DE-C1- 19 529 950
- US-A- 5 749 362

## Description

### BACKGROUND OF THE INVENTION

### Field of the Invention

This invention relates to sensing the position and orientation of an object placed within a living body. More particularly, this invention relates to stabilizing the position and orientation of an intravascular probe within a moving internal organ of a living body.

### Description of the Related Art

A wide range of medical procedures involve placing objects, such as sensors, tubes, catheters, dispensing devices, and implants, within the body. Realtime imaging methods are often used to assist operators in visualizing the object and its surroundings during these procedures. In most situations, however, realtime three-dimensional imaging is not possible or desirable. Instead, systems for obtaining realtime spatial coordinates of the internal object are often utilized.

Many such position sensing systems have been developed or envisioned in the prior art. Some systems involve attaching sensors to the internal object in the form of transducers or antennas, which can sense magnetic, electric, or ultrasonic fields generated outside of the body. For example, U.S. Patent No. 5,983,126, issued to Wittkampf, describes a system in which three substantially orthogonal alternating signals are applied through the subject. A catheter is equipped with at least one measuring electrode, and a voltage is sensed between the catheter tip and a reference electrode. The voltage signal has components corresponding to the three orthogonal applied current signals, from which calculations are made for determination of the three-dimensional location of the catheter tip within the body. Similar methods for sensing voltage differentials between electrodes are proposed by U.S. Patent No. 5,899,860, issued to Pfeiffer. In both of these systems, it is necessary to undertake a separate calibration procedure in order to adjust for discrepancies between the apparent position of the catheter tip as measured and its actual position.

Hybrid catheters are now known that perform ultrasound imaging in conjunction with position sensing. Such devices are disclosed, for example, in U.S. Patent Nos. 6,690,963, 6,716,166 and 6,773,402, which are herein referred to. Medical applications include three-dimensional mapping of a cavity of the body, as well as measurement of chamber wall thickness, wall velocity, and mapping of electrical activity. In medical applications, it is common to acquire maps and images of body organs by different modalities, which are to be interpreted in relationship to one another. An example is correlation of an electro-anatomical map of the heart and an image, such as a three-dimensional ultrasound image. A further system is disclosed in WO 01/89405, which relates to a robot assisted camera guidance system for laparoscopic interventions. An instrument catheter and an imaging catheter are each provided with a position sensing probe, the signals from which are fed to a position decoder, whereafter a manipulating robot is controlled to position the imaging catheter for maintaining the instrument catheter in view. Also known, from WO 97/29682 is a system for the monitoring of a biopsy needle during its introduction to a target site in the body. An external imaging device is provided for obtaining images of a field of view encompassing target tissue or a planned path to the target tissue. A position sensor provided on the biopsy needle allows the imaging device to be directed towards the biopsy needle. The document DE 19529950 relates to a system allowing robotic adjustment of a stereo-laparoscope camera for tracking a surgical instrument in the body. The instrument is detected within images from the laparoscope, and robotic adjustment of the laparoscope position is effected on the basis of the position of the surgical instrument in the field of view of the image.

Commercial electrophysiological and physical mapping systems based on detecting the position of a probe inside the body are presently available. Among them, the Carto-Biosense^{®} Navigation System, available from Biosense Webster Inc., 3333 Diamond Canyon Road Diamond Bar, CA 91765, is a system for automatic association and mapping of local electrical activity with catheter location.

### SUMMARY OF THE INVENTION

Hybrid catheters, for example, catheters having ultrasound transducers and a location sensor provide real-time visualization of anatomical structures and of surgical procedures. The catheter field of view and the resulting ultrasound images have the form of a two-dimensional "fan," which opens outward from the catheter tip and provides a sectional image of the tissue that it intersects. If the location or orientation of the tip is incorrect or unstable, the fan may fail to capture a desired structure or may lose the structure during viewing. Disclosed embodiments of the present invention provide methods and systems for directing and stabilizing the orientation of the ultrasound beam. This is particularly useful in imaging an area in which a surgical procedure is being performed. For example, ultrasound imaging can verify that an ablation catheter is in place and in contact with tissue to be ablated. Subsequent to ablation, ultrasound imaging can confirm that ablation was successful because of the change in echogenicity of the tissue. Stabilization of the catheter using the principles of the present invention ensures that the operator has accurate, near realtime visual feedback related to the target of interest. A catheter having the capabilities just described is sometimes referred to herein as an ultrasound catheter or an ultrasound imaging catheter.

In some aspects of the present invention, convenience of echocardiographic guidance in single operator intracardiac therapeutic procedures is enhanced. By robotically steering an ultrasound catheter to automatically follow the tip of an operative catheter, such as a mapping or ablating catheter, the operator is relieved of the burden of adjusting the imaging catheter to track the mapping or ablation catheter and its target. Realtime visualization of a target site is also enabled during the catheterization procedure, enabling accurate lesion targeting and optimal execution of a therapeutic ablation plan. Other advantages of the invention include monitoring catheter-tissue contact, monitoring the progress of ablation, including detection of bubble and char formation in tissues at the target.

Although the magnetic-based position and orientation sensor in the ultrasound catheter enables the operator to know the catheter position and orientation at all times, it does not by itself guarantee success in holding the catheter stationary in a desired position. Embodiments of the present invention, as claimed hereinafter, solve this problem by using automatic control of the ultrasound catheter to ensure that the catheter is correctly positioned, and oriented toward the target.

WO 01/89405 relates to an operating system for carrying out operation interventions, comprising a laparoscope for visualizing the interventions, said laparoscope being mounted on a robot arm, and a surgical instrument for carrying out the interventions. The laparoscope is guided automatically with a robot arm, using positions of the laparoscope and the surgical instrument detected by position sensors, so that the surgical instrument is always in the field of vision of the laparoscope. It also relates to a method for guiding a laparoscope mounted on a robot arm, used in this operating system.

The invention, is defined by appended claims.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is an illustration of a system for imaging and mapping a heart of a patient in accordance with a disclosed embodiment of the invention;
Fig. 2 schematically illustrates an embodiment of the distal end of s catheter used in the system shown in Fig. 1, in accordance with a disclosed embodiment of the invention;
Fig. 3 is a schematic exploded view of a diagnostic image of the heart, in accordance with a disclosed embodiment of the invention;
Fig. 4 schematically illustrates a control mechanism used by the system shown in Fig. 1 to maneuver an imaging catheter during a medical procedure in accordance with a disclosed embodiment of the invention; and

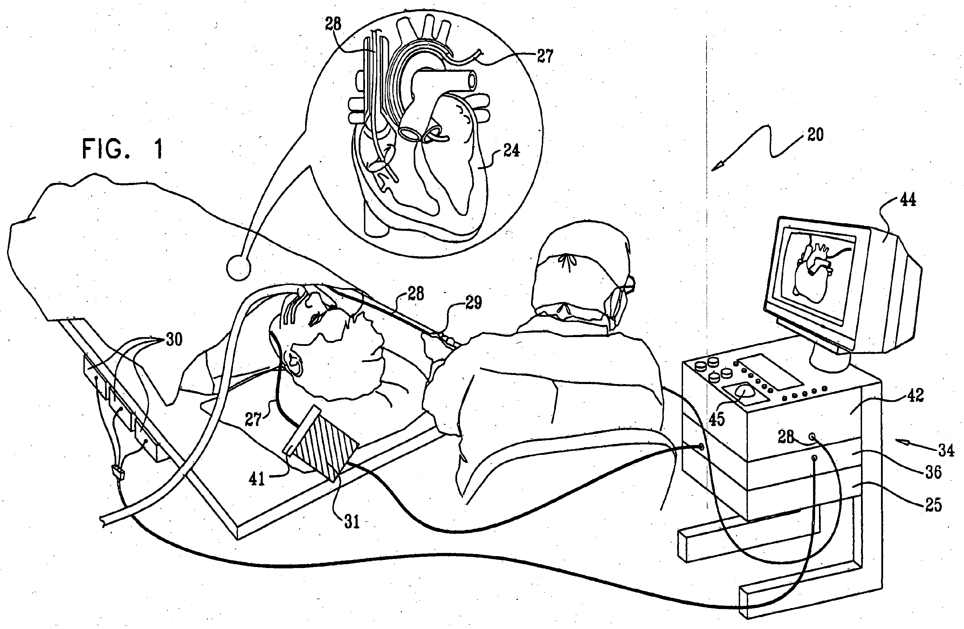

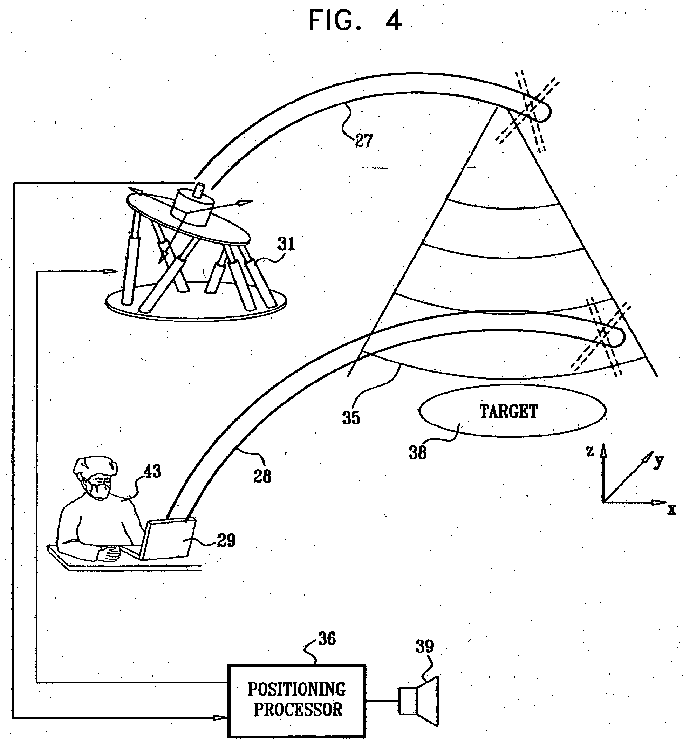

Fig. 4 schematically illustrates a control mechanism used by the system shown in Fig. 1 to maneuver an imaging catheter during a medical procedure in accordance with a disclosed embodiment of the invention; and

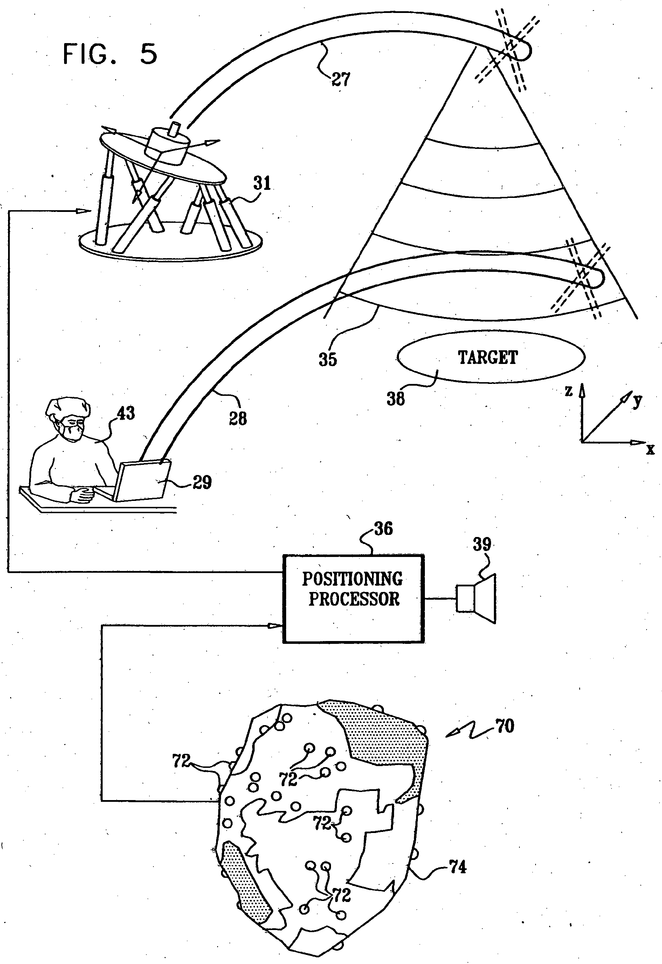

Fig. 5 schematically illustrates a control mechanism used by the system shown in Fig. 1 to maneuver an imaging catheter during a medical procedure in accordance with an alternate embodiment of the invention.

### DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### System Overview

Turning now to the drawings, reference is initially made to Fig. 1, which is an illustration of a system 20 for imaging and mapping a heart 24 of a patient, and which is suitable for performing diagnostic or therapeutic procedures involving the heart 24, in accordance with an embodiment of the present invention. The system comprises a catheter 28, which is percutaneously inserted by an operator 43, who is typically a physician, into a chamber or vascular structure of the heart. The catheter 28 typically comprises a handle 29 for operation of the catheter by the physician. Suitable controls on the handle enable the physician to steer, position and orient the distal end of the catheter as desired to effect a medical procedure. A second catheter 27 is used for imaging the heart, and for determining the position of the catheter 28 in relation to a target, as described below. The catheter 27 has a steering mechanism 41 that is controlled by a robotic manipulator 31, and optionally by the operator 43. The manipulator 31 receives control signals from a positioning processor 36, located in a console 34.

The system 20 comprises a positioning subsystem that measures location and orientation coordinates of the catheter 28. Throughout this patent Application, the term "location" refers to the spatial coordinates of the catheter, and the term "orientation" refers to its angular coordinates. The term "position" refers to the full positional information of the catheter, comprising both location and orientation coordinates.

In one embodiment, the positioning subsystem comprises a magnetic position tracking system that determines the position and orientation of the catheter 28 and the catheter 27. The positioning subsystem generates magnetic fields in a predefined working volume its vicinity and senses these fields at the catheter. The positioning subsystem typically comprises a set of external radiators, such as field generating coils 30, which are located in fixed, known positions external to the patient. The coils 30 generate fields, typically electromagnetic fields, in the vicinity of the heart 24.

In an alternative embodiment, a radiator in the catheter, such as a coil, generates electromagnetic fields, which are received by sensors (not shown) outside the patient's body.

The position sensor transmits, in response to the sensed fields, position-related electrical signals over cables 33 running through the catheter to the console 34. Alternatively, the position sensor may transmit signals to the console over a wireless link. The positioning processor 36 that calculates the location and orientation of the catheter 28 based on the signals sent by a position sensor 32. The positioning processor 36 typically receives, amplifies, filters, digitizes, and otherwise processes signals from the catheter 28. The positioning processor 36 also provides signal input to the manipulator 31 for maneuvering the catheter 27.

Some position tracking systems that may be used for this purpose are described, for example, in U.S. Patents 6,690,963, 6,618,612 and 6,332,089, and U.S. Patent Application Publications 2002/0065455 A1, 2004/0147920 A1, and 2004/0068178 A1. Although the positioning subsystem shown in Fig. 1 uses magnetic fields, the methods described below may be implemented using any other suitable positioning subsystem, such as systems based on electromagnetic fields, acoustic or ultrasonic measurements.

Alternatively, the system 20 can be realized as the above-referenced Carto-Biosense Navigation System, suitably modified to execute the procedures described hereinbelow. For example, the system 20 may employ, *mutatis mutandis,* the catheters disclosed in the above-noted U.S. Patent Nos. 6,716,166 and 6,773,402 in order to acquire ultrasound images for display in near realtime.

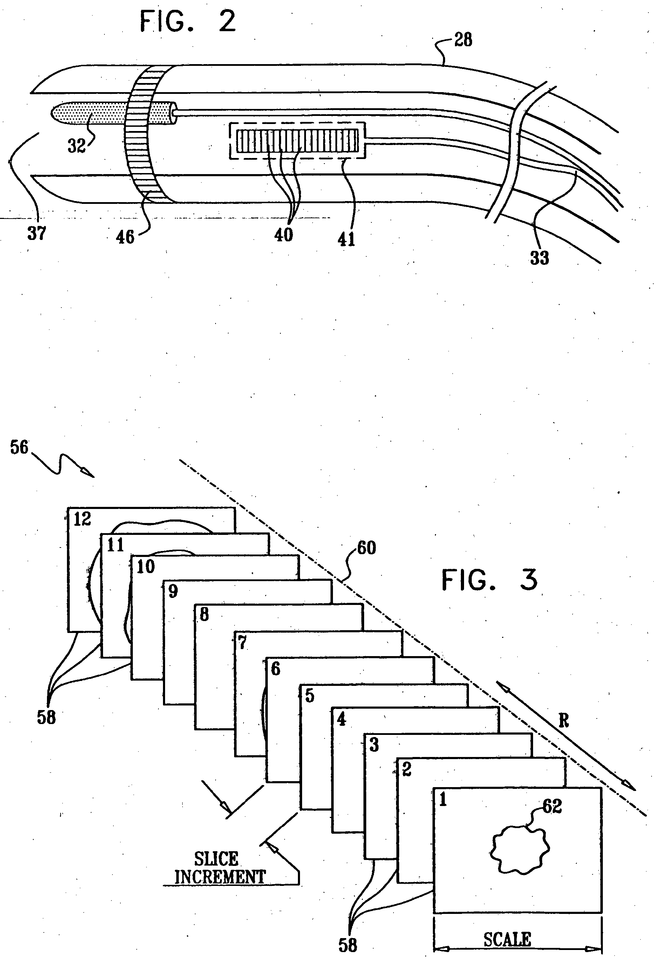

Reference is now made to Fig. 2, which schematically illustrates the distal end of the catheter 28 (Fig. 1), in accordance with a disclosed embodiment of the invention. The fields generated by the field generating coils 30 (Fig. 1) are sensed by the position sensor 32 inside the catheter 28. The catheter 28 also comprises an ultrasonic imaging sensor, which is typically realized as an array of ultrasonic transducers 40. In one embodiment, the transducers are piezo-electric transducers. The ultrasonic transducers are positioned in or adjacent to a window 41, which defines an opening within the body or wall of the catheter. The catheter 28 typically has at least one lumen 37, which can admit a guide wire and guide tube to aid in deployment of a therapeutic device.

The transducers 40 operate as a phased array, jointly transmitting an ultrasound beam from the array aperture through the window 23. Although the transducers are shown arranged in a linear array configuration, other array configurations can be used, such as circular or convex configurations. In one embodiment, the array transmits a short burst of ultrasound energy and then switches to a receiving mode for receiving the ultrasound signals reflected from the surrounding tissue. Typically, the transducers 40 are driven individually in a controlled manner in order to steer the ultrasound beam in a desired direction. By appropriate timing of the transducers, the produced ultrasound beam can be given a concentrically curved wave front, to focus the beam at a given distance from the transducer array. Thus, the system 20 (Fig. 1) uses the transducer array as a phased array and implements a transmit/receive scanning mechanism that enables the steering and focusing of the ultrasound beam, so as to produce two-dimensional ultrasound images.

In one embodiment, the ultrasonic sensor comprises between sixteen and sixty-four transducers 40, preferably between forty-eight and sixty-four transducers. Typically, the transducers generate the ultrasound energy at a center frequency in the range of 5-10 MHz, with a typical penetration depth of 14 cm. The penetration depth typically ranges from several millimeters to around 16 centimeters, and depends upon the ultrasonic sensor characteristics, the characteristics of the surrounding tissue and the operating frequency. In alternative embodiments, other suitable frequency ranges and penetration depths can be used. catheter 28 to an image processor 42 (Fig. 1) in the console 34, which transforms them into two-dimensional, typically sector-shaped ultrasound images. The positioning processor 36 in cooperation with the image processor 42 typically computes or determines position and orientation information, displays real-time ultrasound images, performs three-dimensional image or volume reconstructions. and other functions, which will all be described in greater detail below.

Position sensors and ultrasonic transducers in the catheter 27 (Fig. 1) are similar to those of the catheter 28, except that the transducers of the catheter 27 may be adapted for imaging applications, rather than delivery of therapeutic ultrasound energy to a target.

In some embodiments, the image processor 42 uses the ultrasound images and the positional information to produce a three-dimensional model of a target structure of the patient's heart. The three-dimensional model is presented to the physician as a two-dimensional projection on a display 44.

The distal end of the catheter 28 also comprises at least one electrode 46 for performing diagnostic functions, therapeutic functions or both, such as electro-physiological mapping and radio frequency (RF) ablation. In one embodiment, the electrode 46 is used for sensing local electrical potentials. The electrical potentials measured by the electrode 46 may be used in mapping the local electrical activity at contact points of the endocardial surface. When the electrode 46 is brought into contact or proximity with a point on the inner surface of the heart 24 (Fig. 1), it measures the local electrical potential at that point. The measured potentials are converted into electrical signals and sent through the catheter to the image processor for display as a map reflecting the functional data or activity at each contact point. In other embodiments, the local electrical potentials are obtained from another catheter comprising suitable electrodes and a position sensor, all connected to the console 34. In some applications, the electrode 46 can be used to determine when the catheter is in contact with a valve, since the electrical potentials are weaker there than in the myocardium.

Although the electrode 46 is shown as being a single ring electrode, the catheter may comprise any number of electrodes in any form. For example, the catheter may comprise two or more ring electrodes, a plurality or array of point electrodes, a tip electrode, or any combination of these types of electrodes for performing the diagnostic and therapeutic functions outlined above.

The position sensor 32 is typically located within the distal end of the catheter 28, adjacent to the electrode 46 and the transducers 40. Typically, the mutual positional and orientational offsets between the position sensor 32, electrode 46 and transducers 40 of the ultrasonic sensor are constant. These offsets are used by the positioning processor 36 to derive the coordinates of the ultrasonic sensor and of the electrode 46, given the measured position of the position sensor 32. In another embodiment, the catheter 28 comprises two or more position sensors 32, each having constant positional and orientational offsets with respect to the electrode 46 and the transducers 40. In some embodiments, the offsets (or equivalent calibration parameters) are pre-calibrated and stored in the positioning processor 36. Alternatively, the offsets can be stored in a memory device (such as an electrically programmable read-only memory, or EPROM) fitted into the handle 29 (Fig. 1) of the catheter 28.

The position sensor 32 typically comprises three non-concentric coils (not shown), such as described in U.S. Patent No. 6,690,963, cited above. Alternatively, any other suitable position sensor arrangement can be used, such as sensors comprising any number of concentric or non-concentric coils, Hall-effect sensors or magneto-resistive sensors.

Typically, both the ultrasound images and the position measurements are synchronized with the heart cycle, by gating signal and image capture relative to a body-surface electrocardiogram (ECG) signal or intra-cardiac electrocardiogram. (In one embodiment, the ECG signal can be produced by the electrode 46.) Since features of the heart change their shape and position during the heart's periodic contraction and relaxation, the entire imaging process is typically performed at a particular timing with respect to this period. In some embodiments, additional measurements taken by the catheter, such as measurements of various tissue characteristics, temperature and blood flow measurements, are also synchro-' nized to the electrocardiogram (ECG) signal. These measurements are also associated with corresponding position measurements taken by the position sensor 32. The additional measurements are typically overlaid on the reconstructed three-dimensional model.

In some embodiments, the position measurements and the acquisition of the ultrasound images are synchronized to an internally generated signal produced by the system 20. For example, the synchronization mechanism can be used to avoid interference in the ultrasound images caused by a certain signal. In this example, the timing of image acquisition and position measurement is set to a particular offset with respect to the interfering signal, so that images are acquired without interference. The offset can be adjusted occasionally to maintain interference-free image acquisition. Alternatively, the measurement and acquisition can be synchronized to an externally supplied synchronization signal.

In one embodiment, the system 20 comprises an ultrasound driver 25 that drives the ultrasound transducers 40. One example of a suitable ultrasound driver, which can be used for this purpose is an AN2300™ ultrasound system produced by Analogic Corp. (Peabody, Massachusetts). In this embodiment, the ultrasound driver performs some of the functions of the image processor 42, driving the ultrasonic sensor and producing the two-dimensional ultrasound images. The ultrasound driver may support different imaging modes such as B-mode, M-mode, CW Doppler and color flow Doppler, as are known in the art.

Typically, the positioning processor 36 and image processor 42 are implemented using a general-purpose computer, which is programmed in software to carry out the functions described herein. The software may be downloaded to the computer in electronic form, over a network, for example, or it may alternatively be supplied to the computer on tangible media, such as CD-ROM. The positioning processor and image processor may be implemented using separate computers or using a single computer, or may be integrated with other computing functions of the system 20. Additionally or alternatively, at least some of the positioning and image processing functions may be performed using dedicated hardware.

### Two-Dimensional Anatomic Imaging

Referring again to Fig. 1, using the catheter 27, gated images, e.g., ultrasound images, of the heart are created, and registered with location data of the catheter 28. Suitable registration techniques are disclosed in U.S. Patent No. 6,650,927.

Reference is now made to Fig. 3, which is a schematic exploded view of a diagnostic image 56 of the heart 24 (Fig. 1), in accordance with a disclosed embodiment of the invention. The view is generated using a bullseye rendition technique. The image 56 comprises a stack of parallel slices 58, which are perpendicular to an axis 60. The slices are typically taken at a fixed slice increment along the axis 60. Each slice shows a section 62.

### Three-Dimensional Anatomic Imaging

Referring again to Fig. 1, three-dimensional imaging is described in commonly assigned Application No. 11/115,002 filed on April 26, 2005, entitled *Three-Dimensional Cardiac Imaging Using Ultrasound Contour Reconstruction.* Essentially, three-dimensional image is constructed by combining multiple two-dimensional ultrasound images, acquired at different positions of the catheter 27 into a single three-dimensional model of the target structure. The catheter 27 may operate in a scanning mode, moving between different positions inside a chamber of the heart 24. In each catheter position, the image processor 42 acquires and produces a two-dimensional ultrasound image. In one embodiment, the catheter 27 is side-looking, and a partial three-dimensional reconstruction of the heart is obtained by dithering the catheter, using the manipulator 31, so as to vary its roll angle in an oscillatory manner. Alternatively, the catheter 27 can be dithered so as to vary its pitch or yaw angle. In any case, the result is displayed as a three-dimensional segment of the cardiac chamber, including the catheter 28 and its current target structure.

Alternatively, the catheter 28 is provided with a two-dimensional array of transducers 40 (Fig. 2), which can be phased in order to sweep the beam in an oscillatory manner and thereby obtain different two-dimensional images of the target structure in a planes, while the catheter 28 is held in a fixed position.

### Tracking and Display

Referring again to Fig. 1, during a medical procedure the system 20 can continuously track and display the three-dimensional position of the catheter 28, using the catheter 27 to produce near real-time images of the catheter 28 and its target area. The positioning subsystem of the system 20 repetitively measures and calculates the current position of the catheter 28. The calculated position is stored together with the corresponding slice or slices 58 (Fig. 3). Typically, each position of the catheter 28 is represented in coordinate form, such as a six-dimensional coordinate (X, Y, Z axis positions, and pitch, yaw and roll angular orientations).

The image processor 42 subsequently assigns three-dimensional coordinates to contours of interest, e.g., features identified in the set of images. The location and orientation of the planes of these images in three-dimensional space are known by virtue of the positional information, stored together with the images. Therefore, the image processor is able to determine the three-dimensional coordinates of any pixel in the two-dimensional images. When assigning the coordinates, the image processor typically uses stored calibration data comprising position and orientation offsets between the position sensor and the ultrasonic sensor, as described above.

Alternatively, the system 20 can be used for three-dimensional display and projection of two-dimensional ultrasound images, without reconstructing a three-dimensional model. For example, the physician can acquire a single two-dimensional ultrasound image. Contours of interest on this image can be tagged using the procedures described below. The system 20 can then orient and project the ultrasound image in three-dimensional space.

Reference is now made to Fig. 4, which schematically illustrates a mechanism used by the system 20 (Fig. 1) to effect real-time control of an imaging catheter during a medical procedure in accordance with a disclosed embodiment of the invention. The positioning processor 36 uses signals developed by the position sensor 32 (Fig. 2) to determine the location of the catheter 28, and varies signals that are transmitted to the manipulator 31. The catheter 27 is then automatically maneuvered by the manipulator 31, such that the current location of the catheter 28 is always included in a field of view 35 of the catheter 27. The positioning processor 36 also receives signals from the position sensor (not shown) in the catheter 27 so that it can determine the relative locations of the catheters 27, 28.

Using the information obtained from the catheters 28, 27, the position sensing system determines the current appropriate location and orientation of the catheter 27, and measures any deviations. It then automatically signals the manipulator 31 to execute compensatory maneuvers of the catheter 27. Optionally, an annunciator 39 may audibly or visually cue the operator to override the manipulator 31 and adjust the position of the catheter 27 manually.

Once the target is in proximity with the catheter 28, an enhanced mode of operation is enabled. Using images developed by the image processor 42 (Fig. 1), a target 38 is identified, generally by the operator, but alternatively using information obtained from a knowledge base or a pre-acquired map, as described below. The positioning processor 36 then instructs the manipulator 31 not only to include the catheter 28 in the field of view 35, but also the target 38. The system 20 (Fig. 1) then displays the catheter 28 and the target 38 on the display 44 in a perspective view that is most helpful to the operator. For example, in endoscopic applications, the display 44 can present complementary angular views as requested by the operator.

### Alternative Embodiments outside the scope of the present invention

The techniques described may also be used to keep the ultrasound catheter aimed toward a target that is not equipped with a position sensor. Referring again to Fig. 1, the catheter 27 may be controlled to aim the ultrasound beam continuously toward a landmark in the heart. There are alternative ways of fixing the location and orientation of the ultrasound beam to include the landmark.

The operator 43 indicates fixed reference coordinates on a pre-acquired map. A suitable map can be prepared using the methods described in U.S. Patent 6,226,542, whose disclosure is incorporated herein by reference, Essentially, a processor reconstructs a three-dimensional map of a volume or cavity in a patient's body from a plurality of sampled points on the volume whose position coordinates have been determined. In the case of a moving structure, such as the heart the sampled points are related to a reference frame obtained by gating the imaging data at a point in the cardiac cycle. When acquiring the map, a reference catheter is fixedly positioned in the heart, and the sampled points are determined together with the position of the reference catheter, which is used to register the points.

Reference is now made to Fig. 5, which schematically illustrates a control mechanism used by the system 20 (Fig. 1) to effect real-time tracking and control of an imaging catheter during a medical procedure. Fig. 5 is similar to Fig. 4, except now the positioning processor 36 does not receive signals from the location sensor of the catheter 27. Instead, the position of the catheter 27 is determined automatically by the positioning processor 36 with reference to suitably transformed coordinates of a map 70, which is shown in Fig. 5 as a reconstructed heart volume. The map 70 has a plurality of sampled points 72, which are used to reconstruct a surface 74. A grid (not shown) is adjusted to form the surface 74, in which each point on the grid receives a reliability value indicative of the accuracy of the determination. When the map 70 is displayed for the operator 43, areas of the surface 74 that are covered by relatively less-reliable grid points may be displayed semi-transparently. Alternatively or additionally, different levels of semi-transparency are used together with a multi-level reliability scale.

Alternatively, the map 70 may indicate coordinates of the target, which are then used as points of reference.

The embodiments represented by Fig. 5 may be used to aim the ultrasound catheter toward an important landmark, such as the left atrial appendage or the mitral valve. The purpose of this can be, e.g., to confirm that the area is not being damaged by the medical procedure or that emboli are not developing. As an additional example, the embodiments may be used to confirm the depth of ablation lesions.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. A system (20) for displaying structural information in a body of a living subject, comprising:
an ultrasound imaging catheter (27) adapted for introduction into said body, said ultrasound imaging catheter (27) having a field of view (35) and having a position sensor (32) therein;
an operative catheter (28) adapted for introduction into said body and for effecting a medical procedure on a target structure of said body, said operative catheter (28) having a position sensor (32) therein;
a robotic manipulator (31) operative for maneuvering said ultrasound imaging catheter (27) in said body;
a positioning processor (36) linked to said robotic manipulator (31), said positioning processor (36) being operative responsively to signals from the position sensor (32) of the ultrasound imaging catheter (27) and the position sensor (32) of the operative catheter (28) to determine a position of the operative catheter (28) relative to the ultrasound imagining catheter (27) while effecting the medical procedure, the positioning processor being operative responsively to the current position of the operative catheter (28) to transmit control signals to said robotic manipulator (31) to cause said robotic manipulator (31) to maneuver said ultrasound imaging catheter (27) to maintain a portion of the operative catheter (28) in the field of view (35);
an image processor (42) operative to generate an image of said field of view responsively to image data received from said ultrasound imaging catheter (27);
a display for displaying said image; and **characterised in that**
the system includes an enhanced mode of operation configured to be enabled once a target (38) is in proximity with the operative catheter (28), in which the positioning processor (36) is operative to instruct the robotic manipulator to include the target (38) in the field of view (35) along with the operative catheter (28), wherein the target (38) is identified using images developed by the image processor (42), and displayed on the display (44) in a perspective view.

2. The system (20) according to any preceding claim, wherein said image processor (42) is operative for generating a two-dimensional image of said field of view (35).

3. The system (20) according to any preceding claim, wherein said robotic manipulator is operative to maneuver said ultrasound imaging catheter in an oscillatory motion, and said image processor is operative for generating a plurality of two-dimensional images of said field of view, and said image comprises a three-dimensional image that is constructed by said image processor from said plurality of two-dimensional images.

## Patentansprüche

1. System (20) zum Anzeigen von Strukturinformationen in einem Körper einer lebenden Person, das Folgendes umfasst:
einen Ultraschallabbildungskatheter (27), der für ein Einführen in den Körper ausgelegt ist, wobei der Ultraschallabbildungskatheter (27) ein Sichtfeld (35) besitzt und in sich einen Positionssensor (32) besitzt;
einen betreibbaren Katheter (28), der für ein Einführen in den Körper und zum Ausführen eines medizinischen Vorgangs an einer Zielstruktur des Körpers ausgelegt ist, wobei der betreibbare Katheter (28) in sich einen Positionssensor (32) besitzt;
einen robotergesteuerten Manipulator (31), der betreibbar ist, den Ultraschallabbildungskatheter (27) in dem Körper zu manövrieren;
einen Positionierungsprozessor (36), der mit dem robotergesteuerten Manipulator (31) verbunden ist, wobei der Positionierungsprozessor (36) betreibbar ist, als Reaktion auf Signale von dem Positionssensor (32) des Ultraschallabbildungskatheters (27) und dem Positionssensor (32) des betreibbaren Katheters (28) eine Position des betreibbaren Katheters (28) in Bezug auf den Ultraschallabbildungskatheter (27) zu bestimmen, während der medizinische Vorgang ausgeführt wird, wobei der Positionierungsprozessor betreibbar ist, als Reaktion auf die derzeitige Position des betreibbaren Katheters (28) Steuersignale an den robotergesteuerten Manipulator (31) zu senden, um zu bewirken, dass der robotergesteuerte Manipulator (31) den Ultraschallabbildungskatheter (27) manövriert, um einen Teil des betreibbaren Katheters (28) im Sichtfeld (35) zu behalten;
einen Bildprozessor (42), der betreibbar ist, ein Bild des Sichtfeldes als Reaktion auf von dem Ultraschallabbildungskatheter (27) empfangene Bilddaten zu erzeugen;
eine Anzeige zum Anzeigen des Bildes; und **dadurch gekennzeichnet, dass**
das System eine erweiterte Betriebsart enthält, die konfiguriert ist, dann aktiviert zu werden, wenn ein Ziel (38) in der Nähe des betreibbaren Katheters (28) liegt, wobei der Positionierungsprozessor (36) betreibbar ist, den robotergesteuerten Manipulator anzuweisen, das Ziel (38) zusammen mit dem betreibbaren Katheter (28) in das Sichtfeld (35) einzuschließen, wobei das Ziel (38) unter Verwendung von Bildern, die durch den Bildprozessor (42) entwickelt werden, identifiziert wird und auf der Anzeige (44) in einer perspektivischen Ansicht angezeigt wird.

2. System (20) nach einem vorhergehenden Anspruch, wobei der Bildprozessor (42) betreibbar ist, ein zweidimensionales Bild des Sichtfeldes (35) zu erzeugen.

3. System (20) nach einem vorhergehenden Anspruch, wobei der robotergesteuerte Manipulator betreibbar ist, den Ultraschallabbildungskatheter in einer schwingenden Bewegung zu manövrieren, und der Bildprozessor betreibbar ist, mehrere zweidimensionale Bilder des Sichtfeldes zu erzeugen, und das Bild ein dreidimensionales Bild umfasst, das durch den Bildprozessor aus den mehreren zweidimensionalen Bildern erstellt wird.

## Revendications

1. Système (20) pour afficher des informations structurales dans le corps d'un sujet vivant, le système comprenant :
un cathéter d'imagerie ultrasonique (27) adapté à être introduit dans ledit corps, ledit cathéter d'imagerie ultrasonique (27) possédant un champ de vision (35) et contenant un capteur de position (32) ;
un cathéter opératoire (28) adapté à être introduit dans ledit corps et à effectuer une intervention médicale sur une structure cible dudit corps, ledit cathéter opératoire (28) contenant un capteur de position (32) ;
un manipulateur robotique (31) fonctionnant pour manoeuvrer ledit cathéter d'imagerie ultrasonique (27) dans ledit corps ;
un processeur d'établissement de position (36) relié à audit manipulateur robotique (31), ledit processeur d'établissement de position (36) fonctionnant, en réponse à des signaux provenant du capteur de position (32) du cathéter d'imagerie ultrasonique (27) et du capteur de position (32) du cathéter opératoire (28), pour déterminer une position du cathéter opératoire (28) par rapport au cathéter d'imagerie ultrasonique (27) pendant qu'il effectue l'intervention médicale, le processeur d'établissement de position fonctionnant, en réponse à la position actuelle du cathéter opératoire (28), pour transmettre des signaux de commande audit manipulateur robotique (31) pour amener ledit manipulateur robotique (31) à manoeuvrer ledit cathéter d'imagerie ultrasonique (27) dans le but de maintenir une partie du cathéter opératoire (28) dans le champ de vision (35) ;
un processeur d'image (42) fonctionnant pour générer une image dudit champ de vision en réponse à des données d'image reçues depuis ledit cathéter d'imagerie ultrasonique (27) ;
un affichage pour afficher ladite image ; et
le système étant **caractérisé en ce qu'**il comporte un mode de fonctionnement amélioré configuré pour être activé une fois qu'une cible (38) se trouve à proximité du cathéter opératoire (28), mode dans lequel le processeur d'établissement de position (36) fonctionne pour donner pour instruction au manipulateur robotique d'inclure la cible (38) dans le champ de vision (35) conjointement avec le cathéter opératoire (28), la cible (38) étant identifiée au moyen d'images développées par le processeur d'image (42) et affichée sur l'affichage (44) dans une vue en perspective.

2. Système (20) selon l'une quelconque des revendications précédentes, dans lequel ledit processeur d'image (42) fonctionne pour générer une image à deux dimensions dudit champ de vision (35).

3. Système (20) selon l'une quelconque des revendications précédentes, dans lequel le manipulateur robotique fonctionne pour manoeuvrer ledit cathéter d'imagerie ultrasonique selon un mouvement oscillant, et ledit processeur d'image fonctionne pour générer une pluralité d'images à deux dimensions dudit champ de vision, et ladite image comprend une image à trois dimensions construite par ledit processeur d'image à partir de ladite pluralité d'images à deux dimensions.
